# EUROPÄISCHE PATENTSCHRIFT

(11) **EP 0 597 911 B1**
(45) Veröffentlichungstag und Bekanntmachung des Hinweises auf die Patenterteilung: **24.04.1996**
(21) Anmeldenummer: 92916046.3
(22) Anmeldetag: 22.07.1992
(51) Int. Cl.: B01J 2/06, B01J 2/08, B01J 13/00, B01J 13/08

(54) **VORRICHTUNG ZUR HERSTELLUNG VON ALGINATKUGELN**
PLANT FOR THE PRODUCTION OF SPHERICAL ALGINATE PELLETS
DISPOSITIF DE PRODUCTION DE GLOBULES D'ALGINATE

(30) Priorität: 30.07.1991 DE 4125133
(43) Veröffentlichungstag der Anmeldung: 25.05.1994
(73) Patentinhaber: Nukem GmbH, D-63754 Alzenau (DE)
(72) Erfinder: ALISCH, Gerhard, D-63486 Bruchköbel (DE); BRAUNEIS, Edwin, D-65317 Rodenbach (DE); PIRSTADT, Bernd, D-69493 Hirschberg (DE); IFFLAND, Norbert, D-63579 Freigericht (DE); BRANDAU, Egbert, D-63579 Alzenau (DE)
(74) Vertreter: Stoffregen, Hans-Herbert, Dr. Dipl.-Phys.
(86) Internationale Anmeldenummer: EP9201670
(87) Internationale Veröffentlichungsnummer: WO9302785

(56) Entgegenhaltungen:
- EP-A- 0 268 866
- EP-A- 0 289 648
- EP-A- 0 391 803

## Beschreibung

Die Erfindung bezieht sich auf eine Vorrichtung zur Herstellung von Alginatkugeln nach dem Oberbegriff des Anspruchs 1.

Alginatkugeln werden z. B. als Verdickungsmittel in Emulsionen, in der kosmetischen und der Lebensmittelindustrie, für Klebstoffe, Appreturen oder als Grundsubstanzen für elastische Abdruckmassen für z. B. die Zahnmedizin benötigt. Dabei sind jedoch im Anwendungsfall die Alginatkugeln durchgehärtet oder nur oberflächengehärtet.

Oberflächengehärtete Alginatkugeln werden z. B. in der Kosmetikindustrie und durchgehärtete Alginatkugeln z. B. als Träger für Enzyme benötigt. Um Alginatkugeln im gewünschten Umfang verwenden zu können, sollten diese hinreichend rieselfähig sein und ein enges Kornspektrum aufweisen. Insbesondere sollte auch eine gleichmäßige Geometrie, also eine Kugelform vorliegen.

Nach der FR-A 2 645 439 ist ein Verfahren zur Herstellung von Alginatkugeln bestimmt für die kosmetische Industrie bekannt. Dabei wird eine Alginatlösung einer Düse zugefüht, von der die Alginatlösungen in Tropfenform abgegeben wird, uni anschließend in eine Calcium-Ionenlösung zu fallen. In der Calcium-Ionenlösung befindet sich ein Endlostransportband, von der die Tropfen aufgefangen und sodann aus der Calcium-Ionenlösung heraustransportiert werden.

Da nicht sichergestellt ist, daß die Tropfen während der Fallstrecke in der Calcium-Ionenlösung in hinreichendem Umfang oberflächengehärtet sind, sind die aus der Calcium-Ionenlösung über das Transportband entnommenen Alginatkugeln häufig abgeplattet. Da sich die Tropfen allein durch Abtropfen der Alginatlösung von der Düse ausbilden, ist ferner ein gewünschtes enges Kornspektrum nicht erzielbar.

Aus der EP 0 289 648 A1 ist ein Verfahren zur Herstellung von Kugeln bekannt, die durch Abtropfen aus einer Flüssigkeit und anschließendes Eintropfen in eine Fällösung hergestellt werden. Nach Eintritt in die Fällösung gelangen Tropfen unmittelbar auf den Boden eines die Fällösung aufnehmenden Gefäßes. Hierdurch bedingt besteht die Gefahr, daß eine Abplattung der Tropfen bzw. Kugeln entsteht. Letzteres ist auch durch das Auftreffen auf die Oberfläche der Fällösung möglich.

Um scheibenförmige Partikel herzustellen, erfolgt nach der EP 0 268 866 A2 eine Vertropfung mittels Schwingungsanregung.

Der vorliegenden Erfindung liegt das Problem zugrunde, eine Vorrichtung der eingangs genannten Art zur Herstellung von Alginatkugeln so weiterzubilden, daß unter anderen, Alginatkugeln mit Kugelgeometrie und engem Kornspektrum gewonnen werden und daß die Aushärtung der Alginatkugeln selbst kontrolliert eingestellt werden kann.

Das Problem wird gemäß der Erfindung dadurch gelöst, daß die Vorrichtung zur Zertropfung der von der Düse abgegebenen Alginatlösung einen Schwingungserreger aufweist und daß die Auffangeinrichtung ein von der Ionenlösung einstellbar durchströmbarer Rohrreaktor mit einer Flüssigkeitssäule derart ist, daß die Tropfen während des Durchfließens der Flüssigkeitssäule zumindest in ihrer Oberfläche verfestigbar sind.

Als Schwingungserreger kann ein mechanischer Schwinger, magnetisch-induktiver Schwinger, ein pneumatischer Schwinger, ein piezoelektrischer Umformer oder ein elektro-akustischer Wandler verwendet werden. Dabei kann der Schwingungserreger auf die Düse und/oder auf die Zuführungsleitung und/oder auf den Vorratsbehälter einwirken. Auch besteht die Möglichkeit, die Alginatlösung z. B. mit einem elektroakustischen Wandler unmittelbar zu beschallen bzw. sie mit einem schwingenden Verdränger/Tauchkolben direkt anzuregen, um den aus der Düse austretenden Alginatlösungsstrahl zu gleichmäßigen Tropfen zu zertropfen.

Vorzugsweise ist die Ionenlösung, bei der es sich vorzugsweise um eine CaCl₂-Lösung handelt, mit einem Tensid oder organischen Lösungsmittel versetzt, um-die Oberflächenspannung der Fällösung herabzusetzen.

Um den "Aufprall" der Alginattropfen auf die Fällösungsoberfläche weit herabzusetzen, kann auf der Ionenlösung eine Schaumschicht ausgebildet sein, die selbst eine geschäumte Lösung von Tensid oder organischem Lösungsmittel ist. Die Höhe dieser Schaumschicht beläuft sich vorzugsweise zwischen 5 und 50 mm.

Die Frequenz, die auf die Vorrichtung bzw. die Alginatlösung einwirkt, ist während des Herstellungsprozesses konstant zu halten, wobei vorzugsweise Anregungsfrequenzen zwischen 50 und 20.000 Hz benutzt werden. Die Viskosität der Alginatlösung sollte kleiner als 200 mPa x s sein. Der Durchmesser der Düse sollte schließlich im Bereich zwischen 50 und 3.000 µm liegen.

Mit diesen Parametern lassen sich von der Geometrie gleichmäßig ausgebildete Alginätkugeln mit einem engen Kornspektrum erzeugen, wobei in Abhängigkeit von der Frequenz und des Düsendurchmessers Alginatkugeldurchmesser zwischen 100 und 4.000 µm erreichbar sind.

Weitere Einzelheiten, Vorteile und Merkmale der Erfindung ergeben sich nicht nur aus den Ansprüchen den diesen zu entnehmenden Merkmalen - für sich und/oder in Kombination -, sondern auch aus der nachfolgenden Beschreibung von der Zeichnung zu entnehmenden bevorzugten Ausführungsbeispielen.

Es zeigen:
- Fig. 1: eine Prinzipdarstellung einer Vorrichtung zur Herstellung von Alginatkugeln,
- Fig. 2: ein erstes Ausführungsbeispiel einer Vorrichtung zur Herstellung von Tropfen einer Alginatlösung und
- Fig. 3: eine zweite Ausführungsform einer Vorrichtung zur Herstellung von Tropfen aus einer Alginatlösung.

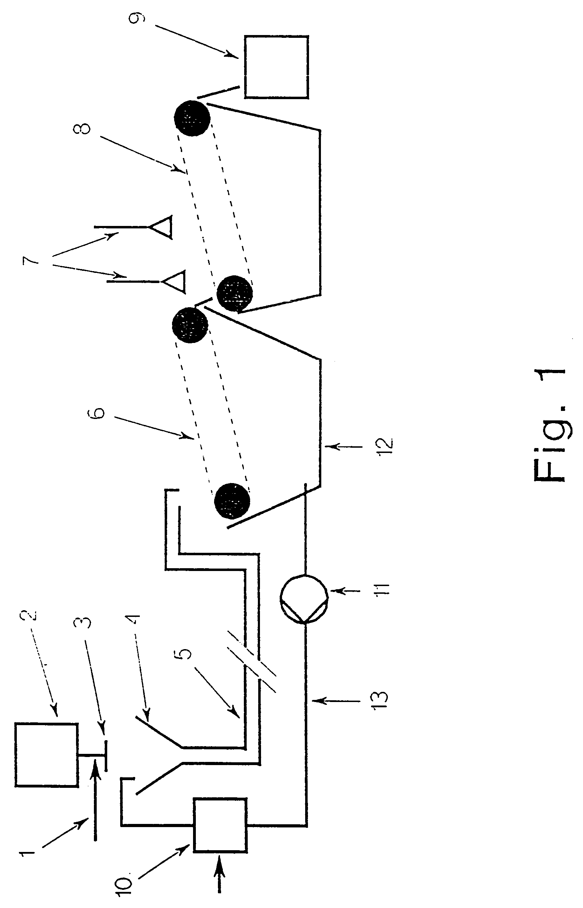

In der Fig. 1 ist mit dem Bezugszeichen (1) eine Zuführung und Dosierung einer Alginatlösung bezeichnet, die über eine Düse (3) an eine Auffangeinrichtung (4) abgegeben ist, in der sich eine Ionenlösung vorzugsweise in Form von 2 % CaCl₂ in VE-Wasser befindet.

Damit die von der Düse (3) abgegebene Alginatlösung zertropft, um also Alginatlösungstropfen reproduzierbarer Größe zu erhalten, ist ein Schwingungserregersystem vorgesehen, das rein prinzipiell angedeutet und mit dem Bezugszeichen (2) versehen ist. Dieses Erregersystem kann unmittelbar auf die Düse (3) einwirken und diese in horizontale oder vertikale Schwingungen versetzen. Auch besteht die Möglichkeit, der Zufühtung (1) Schwingungen aufzuprägen. Alternativ besteht die Möglichkeit, die in einem Vorratshebälter vorhandene Alginatlösung anzuregen. Schließlich kann auch der die Düse (3) verlassende Alginatlösungsstrahl beschallt werden.

Als Schwingungsanreger sind magnetisch-induktive Schwinger, mechanische Schwinger, pneumatische Schwinger, piezoelektrische Umformer und elektro-akustische Wandler denkbar.

Von der Auffangeinrichtung (4), in der auf der Ionenlösung eine Schaumschicht z. B. einer Höhe 5 - 50 mm einer Tensidlösung vorhanden sein kann, fallen die Alginattropfen in der Prinzipdarstellung nach Fig. 1 durch einen Rohrreaktor (5), wobei innerhalb des Rohrreaktors (5) eine freie Beweglichkeit der Alginattropfen zumindest solange erfolgen soll, bis eine Oberflächenverfestigung erfolgt ist. Unter diesen Bedingungen weisen die Alginattropfen die gewünschte Kugelgeometrie auf, so daß ein Zusammenstoß mit anderen Alginattropfen bzw. -kugeln bzw. mit den Wandungen des Rohrreaktors (5) zu keiner Verformung mehr führt.

Nach Verlassen des Rohrreaktors werden die Alginatkugeln gewünschter Verfestigung auf ein Siebband (6) abgegeben, von dem die an den Alginatkugeln verbleibende Calciumchlorid-Lösung abtropft. Von dem Siebband (6) gelangen die Alginatkugeln auf ein Siebband (8), auf dem sie mittels Waschwasserdüsen (7) gewaschen werden. Von dem Siebband (8) werden die Alginatkugeln als fertige Produkte in einer Auffangeinrichtung (9) gesammelt und sodann dem gewünschten Einsatz zugeführt.

Durch den Rohrreaktor (5) fließt die Ionenlösung mit einer gewüschten Geschwindigkeit. Die Verweilzeit der Alginattropfen bzw. -kugeln in der Ionenlösung wird durch die Geschwindigkeit der Ionenlösung und durch die Länge des Rohrreaktors bestimmt. Diese sind somit im gewünschten Umfang, und zwar reproduzierbar, verfestigbar. Die Ionenlösung wird mittels einer Pumpe (11) in einem Kreislauf geführt, die eine Verbindung (13) zwischen einem unterhalb des Siebbandes (6) vorhandenen Auffangebehälter (12) und der Auffangeinrichtung (4) einschließt. In der Verbindung oder Leitung (13) befindet sich des weiteren eine Durchfluß-Konzentrationssteuetung (10) für die Ionenlösung.

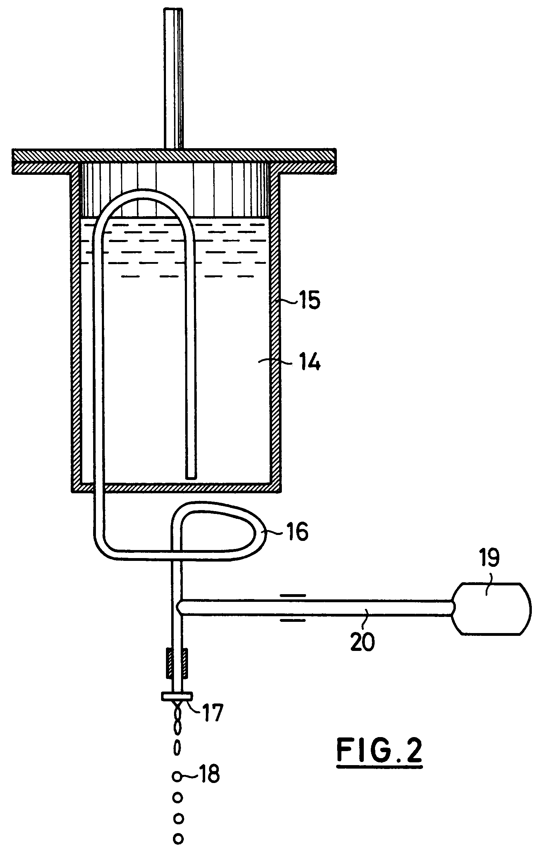

In Fig. 2 ist ein Ausschnitt einer Vorrichtung zur Herstellung von Alginatkugeln dargestellt, und zwar derjenige, durch den die Tropfen aus der Alginatlösung hergestellt werden. Die Alginatlösung (14) befindet sich in einem Vorratsbehältnis (15), von dem die Alginatlösung (14) über eine Zuführungsleitung (16) einer Düse (17) zugeführt wird, von der aus die Alginatlösung (14) in Form von Tropfen (18) schwerkraftbedingt herabfällt. Dabei erkennt man, daß unmittelbar hinter der Düse (17) die Tropfen eine langgestreckte Form aufweisen, die sich auf Grund der Oberflächenspannung der Alginatlösung nach einer Fallstrecke in eine Kugelform ändert.

Mit Hilfe eines Vibrationsgenerators (19), der über eine starre Verbindung (20) mittelbar oder unmittelbar mit der Düse (17) verbunden ist, wird eine Schwingung erzeugt, durch die ein Zertropfen der die Düse (17) verlassenden Alginatlösung erfolgt, d. h. in dem die Düse (17) verlassenden Flüssigkeitsstrahl werden rotationssymmetrische Einschnürungen erzeugt und verstärkt, so daß ein Zerfallen in gleichmäßige Tropfen erfolgt.

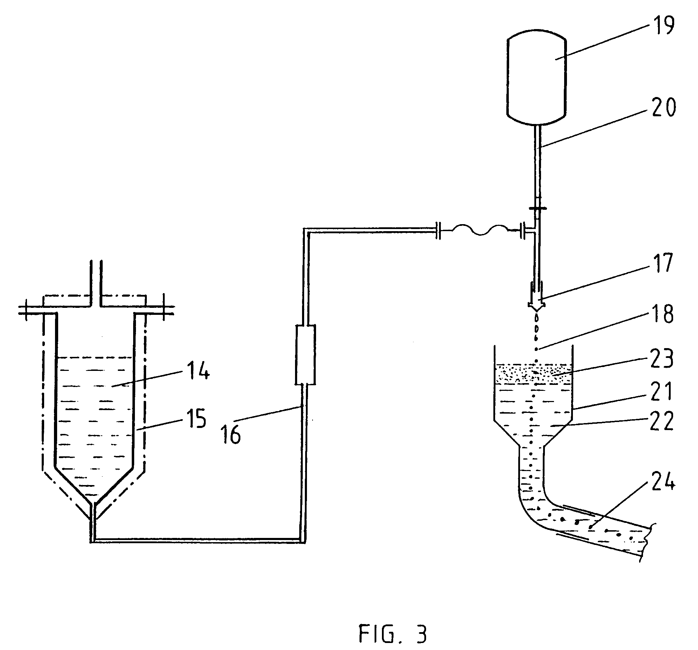

Kann nach dem Ausführungsbeisplel der Fig. 2 die Alginatlösung (14) schwerkraftbedingt der Düse (17) zugeführt werden, so ist nach dem Ausführungsbeispiel der Fig. 3 eine Druckförderung vorgesehen. Ansonsten zeigt der Aufbau der Fig. 3 einen der Fig. 2 entsprechenden. Daher sind auch gleiche Elemente mit gleichen Bezugszeichen versehen.

Ergänzend ist eine Auffangeinrichtung (21) dargestellt, in der z. B. eine Calciumchlorid-Lösung vorhanden ist bzw. durch diese strömt. Oberhalb des Flüssigkeitsspiegels der Calciumchlotid-Lösung befindet sich eine Schaumschicht (23) einer Tensidlösung, durch die Tropfen (18) "abgebremst" werden. Hierdurch ergibt sich der Vorteil, daß beim Auftreffen der Tropfen (18) auf die Flüssigkeitsoberfläche der Calciumchlorid-Lösung ein unerwünschtes Abplatten weitgebend vermieden wird.

Die durch die Lösung fallenden bzw. geförderten Tropfen (18) werden durch Reaktion mit der Calciumchlorid-Lösung zunächst außenseitig verfestigt, so daß sich innerhalb der Chlorid-Lösung (22) Alginatkugeln (24) gewünschter Verfestigung befinden, wobei erwähntermaßen die Verfestigung von der Verweilzeit der Alginatkugeln in der Calciumchlorid-Lösung (22) abhängig ist.

Der die Calciumchlorid- oder auch Fällösung aufnehmende Reaktor (21) kann - wie beim Ausführungsbeispiel der Fig. 1 - ein Rohrreaktor sein, mit dem die exakte Verweilzeit der Alginatkugeln (24) in der Fällösung durch Variieren der Reaktorlänge und der Strömungsgeschwindigkeit der Fällösung eingestellt werden kann, so daß Alginatkugeln (24) erzeugt werden, die eine gewünschte Verfestigung, also z. B. nur in der Oberfläche gehärtet oder voll ausgehärtet sein können.

Bei dem Reaktor kann es sich auch um einen Batch-Reaktor handeln, der gegebenenfalls einen Rührer aufweist, insbesondere dann, wem die Alginatkugeln (24) vollständig ausgehärtet werden sollen.

Zu der Fällösung ist noch zu bemerken, daß dieser Tensid oder organisches Lösungsmittel zugesetzt sein kann, um die Oberflächenspannung zu verringern Auch kann - wie anhand der Fig. 3 beschrieben - auf der Fällösung (22) ein Tensidschaum oder Schaum organischen Lösungsmittels vorhanden sein. Es besteht auch die Möglichkeit, die Fällösung (22) von einer Aufnahmeeinrichtung mit Überlaufrinne aufzunehmen.

Bei der verwendeten Düse zum Zertropfen der Alginatlösung kann es sich um eine Vollstrahldüse unterschiedlicher Materialien handeln Auch besteht die Möglichkeit, eine Düsenplatte, also eine solche mit einer Vielzahl von Düsen zu verwenden.

Die bei dem erfindungsgemäßen Verfahren benutzte Alginatlösung sollte eine Viskosität kleiner 200 Mpa x s aufweisen. Die Anregungsfrequenz, mittels der das Zertropfen der aus der Düse heraustretenden Lösung erfolgt, sollte zwischen 50 und 20.000 Hz liegen. Die Düsendurchmesser selbst können im Bereich zwischen 50 und 3.000 µm liegen. Bei Berücksichtigung dieser Parameter lassen sich Alginatkugelndurchmesser im Bereich zwischen 100 und 4.000 µm gewinnen, die eine nahezu exakte Kugelform aufweisen. Die bei jeweils gleichen Parametern hergestellten Alginatkugeln weisen dabei ein sehr enges Kornspektrum auf.

Anhand nachstehender Beispiele ergeben sich weitere Vorteile und Merkmale der Erfindung, die - jeweils für sich oder in Kombination - als erfinderisch anzusehen sind.

### Beispiel 1:

In einem Vorratsbehältnis (15) befindet sich eine Alginatlösung, die der Düse (17) mit einem Durchmesser von 280 µm zugeführt wird, die ihrerseits mit einer Frequenz von 2.100 Hz in Schwingung versetzt wird. Die zertropfte Alginatlösung fällt in eine Celcium-Ionenlösung, und zwar in 2 % CaCl₂ in VE-Wasser. Die Alginattropfen bzw. -kugeln verweilen in der Fällösung 30 Minuten, wodurch eine vollständige Aushärtung erfolgt. Der Durchmesser der gewonnenen Kugeln beträgt 500 µm bei einer Standardabweichung von ca. 1 %. Auf der Fallösung selbst befand sich kein Schaum.

### Beispiel 2:

Um oberflächenverhärtete Alginatkugeln zu gewinnen, wird ein Rohrreaktor benutzt, in dem sich eine Fällösung in Form von 0,35 % CaCl₂ plus 0,05 % Tensid und VE-Wasser befindet. Die verwendete Düse, mittels der die Alginatlösung zertropft wird, weist einen Durchmesser von 900 µm auf. Die Schwingungsfrequenz beträgt 155 Hz. Die Alginatkugeln verweilen in der Fällösung 1 Minute. Als Ergebnis werden Alginatkugeln, deren Oberfläche gehärtet ist, mit einem Durchmesser von 1.700 µm gewonnen. Die Standardabweichung beträgt auch im vorliegenden Fall 1 %.

### Beispiel 3:

Es wird gleichfalls ein Rohrreaktor benutzt, in dem sich eine Fällösung nachstehender Zusammensetzung befindet: 0,26 % CaCl₂ plus 0,05 % Tensid in VE-Wasser. Die verwendete Düse weist einen Durchmesser von 1925 µm auf. Die Frequenz beträgt 50 Hz. Auf der Fallösung befindet sich ein Tensidschaum in Höhe von in etwa 20 mm. Die Alginatkugeln verweilen in der Fällösung 1,5 Minuten. Als Ergebnis erhält man oberflächengehärtete Alginatkugeln mit Durchmessern von 3.400 µm. Standardabweichung beträgt 1 %.

### Beispiel 4:

Ein dem Beispiel 3 entsprechender Verfahrensablauf erfolgt, wobei jedoch anstelle von 0,05 % Tensid in CaCl₂-Lösung 8 % Isopropylalkohol hinzugegeben wird. Auch die so hergestellten Alginatkugeln weisen gewünschte Eigenschaften hinsichtlich der Oberflächenhärtung und der Durchmesser (3.400 µm bei einer Standardabweichung von 1 %) auf.

## Patentansprüche

1. Vorrichtung zur Herstellung von Alginatkugeln aus Tropfen einer Alginatlösung (14) umfassend ein Vorratsbehälter (15) für die Alginatlösung, zumindes eine Düse (3, 17), der gegebenenfalls über eine Zuführungsleitung (16, 20) die Alginatlösung zuführbar ist, ein eine Ionenlösung enthaltende Auffangeinrichtung (4, 21) für die voll der Düse herabfallenden Tropfen sowie zumindest eine Einrichtung zur Entnahme der Alginatkugeln aus der Auffangeinrichtung (6, 7, 8, 9),
**dadurch gekennzeichnet,**
daß die Vorrichtung zur Zertropfung der von der Düse (3, 17) abgegebenen Alginatlösung (14) einen Schwingungserreger (2, 19) aufweist und daß die Auffangeinrichtung (4, 5, 21) ein von der Ionenlösung (22) einstellbar durchströmbarer Rohrreaktor mit einer Flüssigkeitssäule derart ist, daß die Tropfen (18) während des Durchfließens der Flüssigkeitssäule zumindest in ihrer Oberfläche verfestigbar sind.

2. Vorrichtung nach Anspruch 1,
**dadurch gekennzeichnet,**
daß der Schwingungserreger ein mechanischer oder magnetisch-induktiver Schwinger, ein pneumatischer Schwinger, ein piezoelektrischer Umformer oder ein elektro-akustischer Wandler oder ein schwingender Verdränger/Tauchkolben ist.

3. Vorrichtung nach Anspruch 1 oder 2,
**dadurch gekennzeichnet,**
daß der Schwingungserreger (2, 19) auf die Düse (3,17) und/oder auf die Zuführungsleitung (1, 16) und/oder auf den Vorratsbehälter (15) oder auf den Düsen-Innenraum einwirkt.

4. Vorrichtung nach Anspruch 1,
**dadurch gekennzeichnet,**
daß die Alginatlösung (14) beschallbar ist.

5. Vorrichtung nach zumindest einem der vorhergehenden Ansprüche,
**dadurch gekenzeichnet**,
daß die Ionenlösung Ca²⁺-Ionen oder ein oder mehrere andere Ionen enthält, die mit Alginat eine schwerlösliche Verbindung bilden.

## Claims

1. Apparatus for producing spherical alginate pellets from droplets of an alginate solution (14), comprising a reservoir container (15) for the alginate solution; at least one nozzle (3, 17) to which alginate solution can be fed as applicable via a feed line (16, 20); a collection device (4, 21), containing an ion solution for the droplets failing from the nozzle; and at leat one device for removing the alginate pellets from the collection device (6, 7, 8, 9),
**wherein**
the apparatus for dropletizing the alginate solution (14) delivered by the nozzle (3, 17) has a vibration exciter (2, 19); and the collection device (4, 5, 21) is a tubular reactor through which the ion solution (22) can flow in an adjustable manner with a liquid column of a length such that the droplets (18) cap be consolidated at least on their surface while flowing through the liquid column.

2. Apparatus according to Claim 1,
**wherein**
the vibration exciter is a mechanical or magnetic-induction vibrator, a pneumatic vibrator, a piezoelectric converter or electroacoustic converter, or a vibrating displacer/plunger.

3. Apparatus according to Claim 1 or 2,
**wherein**
the vibration exciter (2, 19) acts on the nozzle (3, 17) and/or on the feed line (1, 16) and/or on the reservoir container (15) or on the interior of the nozzle.

4. Apparatus according to Claim 1,
**wherein**
the alginate solution (14) can be acoustically irradiated.

5. Apparatus according to at least one of the preceding claims,
**wherein**
the ion solution contains Ca²⁺ ions or one or more other ion species that form a poorly soluble compound with alginate.

## Revendications

1. Installation de fabrication de billes d'alginate à partir de gouttes d'une solution d'alginate (14), comprenant un réservoir d'alimentation (15) pour la solution d'alginate, au moins un ajutage (3, 17) auquel on peut éventuellement amener la solution d'alginate par l'intermédiaire d'une conduite d'amenée (16, 20), un dispositif de réception contenant une solution d'ions (4, 21) pour les gouttes tombant de l'ajutage, ainsi qu'au moins un dispositif de prélèvement des billes d'alginate hors du dispositif de réception (6, 7, 8, 9), caractérisé en ce que l'installation de transformation en gouttes de la solution d'alginate (14) libérée par l'ajutage (3, 17) présente un excitateur d'oscillations (2, 19) et en ce que le dispositif de réception (4, 5, 21) est un réacteur tubulaire qui peut être parcouru de manière réglable par la solution d'ions (22) avec une colonne de liquide telle que les gouttes (18) sont solidifiables au moins à leur surface au cours de leur passage à travers la colonne de liquide.

2. Installation suivant la revendication 1, caractérisé en ce que l'excitateur d'oscillation est un oscillateur magnéto-inductif ou mécanique, un oscillateur pneumatique, un convertisseur piézo-électrique, ou un transformateur électro-acoustique, ou un piston plongeur/repousseur oscillant.

3. Installation suivant la revendication 1 ou 2, caractérisée ne ce que l'excitateur d'oscillations (2, 19) agit sur l'ajutage (3, 17) et/ou sur la conduite d'amenée (1, 16) et/ou sur le réservoir d'alimentation (15), ou sur l'espace interne de l'ajutage.

4. Installation suivant la revendication 1, caractérisée en ce que la solution d'alginate (14) peut être traitée aux ultrasons.

5. Installation suivant au moins l'une des revendications précédentes, caractérisée en ce que la solution d'ions contient des ions Ca²⁺ ou un ou plusieurs autres ions, qui forment un composé difficilement soluble avec l'alginate.
